# EUROPÄISCHE PATENTANMELDUNG

(11) **EP 0 982 289 A2**
(43) Veröffentlichungstag der Anmeldung: **01.03.2000**
(21) Anmeldenummer: 99116118.3
(22) Anmeldetag: 18.08.1999
(51) Int. Cl.: C07C 57/07

(54) **Verfahren zur kontinuerlichen Gewinnung von (Meth)acrylsäure**

(30) Priorität: 26.08.1998 DE 19838817
(71) Anmelder: BASF AKTIENGESELLSCHAFT, 67056 Ludwigshafen (DE)
(72) Erfinder: Machhammer, Otto, Dr., 68163 Mannheim (DE); Haupt, Susanne, Dr., 63069 Offenbach (DE); Schliephake, Volker, Dr., 67105 Schifferstadt (DE); Schröder, Jürgen, Dr., 67071 Ludwigshafen (DE)
(74) Vertreter: Isenbruck, Günter, Dr.

(57) **Zusammenfassung**

Verfahren zur kontinuierlichen Gewinnung von (Meth)acrylsäure aus dem (Meth)acrylsäure, Leichtsieder, Mittelsieder und Schwersieder enthaltenden Reaktionsgas einer katalytischen Gasphasenoxidation durch
(I) Quenchen des Reaktionsgases durch Siedekühlung mit einem hochsiedenden organischen Lösungsmittel,
(II) Abtrennung der (Meth)acrylsäure aus dem gequenchten Reaktionsgas durch Absorption in das hochsiedende organische Lösungsmittel,
(III) Auftrennung des mit (Meth)acrylsäure beladenen organischen Lösungsmittels in einen ersten Teilstrom (IIIA), der überwiegend (Meth)acrylsäure enthält sowie in einen zweiten Teilstrom (IIIB), der überwiegend das Lösungsmittel enthält, wobei
(IV) Teilstrom IIIB mit Inertgas frei an (Meth)acrylsäure gestrippt wird,
(V) das gereinigte Lösungsmittel aus Teilstrom IIIB in die Absorptionsstufe II zurückgeführt wird und
(VI) wobei (Meth)acrylsäure aus Teilstrom IIIA destillativ gewonnen wird, wobei alle in Stufe VI anfallenden flüssigen Restströme in die Quenchstufe I zurückgeführt werden.

## Beschreibung

Die Erfindung betrifft ein Verfahren zur kontinuierlichen Gewinnung von (Meth)acrylsäure durch Absorption von (Meth)acrylsäure aus den Reaktionsgasen einer katalytischen Gasphasenoxidation. Der Begriff (Meth)acrylsäure steht im folgenden für die Substanzen Acrylsäure und/oder Methacrylsäure.

(Meth)acrylsäure wird überwiegend durch katalytische Gasphasenoxidation geeigneter Ausgangsstoffe, insbesondere von Propen und/oder Acrolein im Falle der Acrylsäure bzw. von Isobuten und/oder Methacrolein im Falle der Methacrylsäure, hergestellt.

Zur Abtrennung der (Meth)acrylsäure aus den Reaktionsgasen der katalytischen Gasphasenoxidation sind eine Reihe von Möglichkeiten bekannt, darunter auch die Abtrennung durch Absorption in ein Lösungsmittel.

Aus DE-B 21 36 396 ist bekannt, die Acrylsäure aus den bei der katalytischen Oxidation von Propen bzw. Acrolein erhaltenen Reaktionsgasen durch Gegenstromabsorption mit einem Gemisch aus 75 Gew.-% Diphenylether und 25 Gew.-% Diphenyl abzutrennen. Weiterhin ist aus DE-A 24 49 780 das Abkühlen des heißen Reaktionsgases durch Teilverdampfen des Lösungsmittels in einem Direktkondensator (Quenchapparat) vor der Gegenstromabsorption bekannt. Problematisch ist hierbei sowie bei weiteren Verfahrensschritten, insbesondere bei der destillativen Reinigung der (Meth)acrylsäure, der Anfall von Feststoffen in den Apparaten, der die Anlagenverfügbarkeit reduziert. Gemäß DE-A 43 08 087 kann im Fall der Acrylsäure dieser Feststoffanteil reduziert werden, indem man dem relativ unpolaren Lösungsmittelgemisch aus Diphenylether und Diphenyl ein polares Lösungsmittel wie Dimethylphthalat in einer Menge von 0,1 bis 25 Gew.-% zufügt; dadurch erhöht sich das Aufnahmevermögen des Lösungsmittelgemisches für die schmutzbildenden Stoffe. Mit steigender Polarität nimmt das Lösungsmittel jedoch zunehmende Mengen an Wasser mit auf; außerdem führt dies zu erhöhten Lösungsmittelverlusten über das Sauerwasser.

In Gegenwart von Lösungsmitteln bildet die Polyacrylsäure im Bereich höherer Temperaturen, wie sie bei der Gewinnung von (Meth)acrylsäure nach dem gattungsgemäßen Verfahren, insbesondere am untersten Sammelboden der Absorptionskolonne, im Abtriebs- und Sumpfteil der Destillationskolonne sowie in den Wärmetauschern auftreten, einen an der Oberfläche der Apparate fest haftenden Schmutz, der nur mit Laugen gelöst werden kann. Analysen haben gezeigt, daß der Schmutz aus einer Mischung aus ca. 10 bis 50 Gew.-% Poly(meth)acrylsäure, Rest Lösungsmittel, besteht.

Es ist daher Aufgabe der Erfindung, die Verschmutzungsanfälligkeit in sämtlichen Apparaten, insbesondere den Anfall von nur lauge-löslichen Verschmutzungen weitgehend zu vermeiden, und somit die Anlagenverfügbarkeit und die Wirtschaftlichkeit des Verfahrens zur Gewinnung der (Meth)acrylsäure zu verbessern.

Die Erfindung geht aus von einem Verfahren zur kontinuierlichen Gewinnung von (Meth)acrylsäure aus dem (Meth-)acrylsäure, Leichtsieder, Mittelsieder und Schwersieder enthaltenden Reaktionsgas einer katalytischen Gasphasenoxidation durch
(I) Quenchen des Reaktionsgases durch Siedekühlung mit einem hochsiedenden organischen Lösungsmittel,
(II) Abtrennung der (Meth)acrylsäure aus dem gequenchten Reaktionsgas durch Absorption in das hochsiedende organische Lösungsmittel,
(III) Auftrennung des mit (Meth)acrylsäure beladenen organischen Lösungsmittels in einen ersten Teilstrom (IIIA), der überwiegend (Meth)acrylsäure enthält sowie in einen zweiten Teilstrom (IIIB), der überwiegend das Lösungsmittel enthält.
   Das Verfahren ist dann dadurch gekennzeichnet, daß
(IV) Teilstrom IIIB mit Inertgas frei an (Meth)acrylsäure gestrippt wird,
(V) das gereinigte Lösungsmittel aus Teilstrom IIIB in die Absorptionsstufe II zurückgeführt wird und daß
(VI) (Meth)acrylsäure aus Teilstrom IIIA destillativ gewonnen wird, wobei alle in Stufe VI anfallenden flüssigen Restströme in die Quenchstufe I zurückgeführt werden.

In bevorzugter Weise übersteigt die Temperatur in jeder Verfahrensstufe nicht 155°C, insbesondere nicht 140°C, besonders bevorzugt nicht 120°C.

Der Begriff flüssige Restströme bezeichnet vorliegend alle im Verfahren anfallenden flüssigen Ströme außer dem Hauptproduktstrom, beispielsweise die im folgenden näher beschriebenen Teilströme (a) und (c).

Es wurde gefünden, daß bei der destillativen Gewinnung der (Meth)acrylsäure (Stufe VI) Oligomere entstehen, die über die flüssigen Restströme bislang in vorgeschaltete Apparate verschleppt wurden. Indem erfindungsgemäß die Rückführung der flüssigen Restströme aus der destillativen Gewinnung (Stufe VI) in den flüssigen Lösungsmittelkreis vermieden wird, kann die Verschmutzung der vorgeschalteten Apparate weitgehend verhindert werden. Der Anfall an Oligomeren und somit der Feststoffanfall in den der Verfahrensstufe VI vorgeschalteten Apparaten ist beim erfindungsgemäßen Verfahren geringer; daher können die nach den herkömmlichen Verfahren bislang in den Verfahrensstufen II und IV notwendigen Dual-Flow- bzw. Ventilböden durch höher hydrodynamisch belastbare Einbauten, beispielsweise Füllkörper oder Packungen, ausgetauscht werden.

Die weitgehende Vermeidung von Verschmutzung durch die erfindungsgemäße Stromführung hat weitreichende wirtschaftliche Konsequenzen: insbesondere können in den Apparaten Elemente eingesetzt werden, wie Füllkörper oder strukturierte Packungen, die eine größere hydrodynamische Belastbarkeit, jedoch auch eine höhere Verschmutzungsanfälligkeit gegenüber beispielsweise Dual-Flow- oder Ventilböden aufweisen, die nach dem bekannten Verfahren zur Abtrennung von (Meth)acrylsäure aus Gemischen in einem hochsiedenden Lösungsmittel wegen ihrer geringeren Verschmutzungsanfälligkeit eingesetzt werden. Neuanlagen können somit bei gleicher Produktionsmenge mit kleineren Trennapparaten, insbesondere Kolonnen, dimensioniert werden, bzw. wird bei bestehenden Anlagen die Produktionsmenge nach dem erfindungsgemäßen Verfahren erhöht.

Als hochsiedend werden vorliegend Lösungsmittel bezeichnet, deren Siedepunkt höher ist als der Siedepunkt des jeweils angestrebten Hauptprodukts (ca. 141°C für Acrylsäure bzw. ca. 161°C für Methacrylsäure, jeweils bei Normaldruck).

Ausgangsgemische für das vorliegende Verfahren sind die Reaktiongase aus der katalytischen Gasphasenoxidation von C₃-Alkanen, -Alkenen, -Alkanolen und/oder C₃-Alkanen, -Alkenen, -Alkanolen und/oder -Alkanalen oder Vorstufen davon zu Methacrylsäure. Das Verfahren wird im folgenden für Acrylsäure beschrieben, es gilt jedoch analog auch für die Methacrylsäure.

Besonders vorteilhaft ist die katalytische Gasphasenoxidation von Propen und/oder Acrolein zu Acrylsäure in Luft oder molekularem Sauerstoff nach bekannten Verfahren, insbesondere wie sie in den oben genannten Druckschriften beschrieben sind. Vorzugsweise wird hierbei bei Temperaturen zwischen 200 und 450°C und ggf. erhöhtem Druck gearbeitet. Vorzugsweise werden als heterogene Katalysatoren oxidische Mehrkomponenten-Katalysatoren aufder Basis der Oxide von Molybdän, Bismut und Eisen in der 1. Stufe (Oxidation von Propen zu Acrolein) und der Oxide von Molybdän und Vanadium in der 2. Stufe (Oxidation von Acrolein zu Acrylsäure) eingesetzt. Wird Propan als Ausgangsstoff verwendet, so kann dieses zu einem Propen/Propan-Gemisch umgesetzt werden durch katalytische Oxidehydrierung, wie in US-5 510 558 beschrieben oder durch homogene Oxidehydrierung, entsprechend zum Beispiel der EP-A-0 253 409. Bei Einsatz eines Propen-/Propan-Gemischs wirkt Propan als Verdünnungsgas. Geeignete Propen-/Propan-Gemische sind auch Raffineriepropen (70% Propen und 30% Propan) oder Crackerpropen (95% Propen und 5% Propan). Grundsätzlich können Propen-/Propan-Gemische aus Sauerstoff und Stickstoff jeder Zusammensetzung zu Acrolein und Acrylsäure oxidiert werden.

Die Umsetzung von Propen zu Acrylsäure ist stark exotherm. Das Reaktionsgas, das neben den Edukten und Produkten vorteilhafterweise ein inertes Verdünnungsgas, z.B. Kreisgas (siehe unten), Luftstickstoff, einen oder mehrere gesättigte C₁- bis C₆-Kohlenwasserstoffe, insbesondere Methan und/oder Propan und/oder Wasserdampf enthält, kann daher nur einen kleinen Teil der Reaktionswärme aufnehmen. Obwohl die Art der verwendeten Reaktoren an sich keiner Beschränkung unterliegt, werden meist salzbadgekühlte Rohrbündelwärmetauscher verwendet, die mit dem Oxidationskatalysator gefüllt sind, da bei diesen die bei der Reaktion freiwerdenden Wärme sehr gut durch Konvektion und Strahlung an die gekühlten Rohrwände abgeführt werden kann.

Bei der katalytischen Gasphasenoxidation wird nicht reine Acrylsäure, sondern ein gasförmiges Gemisch erhalten, das neben der Acrylsäure als Nebenkomponenten im wesentlichen nicht umgesetztes Acrolein und/oder Propen, Wasserdampf, Kohlenmonoxid, Kohlendioxid, Stickstoff, Propan, Sauerstoff, Essigsäure, Propionsäure, Formaldehyd, weitere Säuren und Aldehyde, Maleinsäure bzw. Maleinsäureanhydrid enthalten kann. Üblicherweise enthält das Reaktionsproduktgemisch, jeweils bezogen auf das gesamte Reaktionsgemisch, 1 bis 30 Gew.-% Acrylsäure, 0,05 bis 1 Gew.-% Propen und 0,05 bis 1 Gew.-% Acrolein, 0,05 bis 10 Gew.-% Sauerstoff, 0,05 bis 2 Gew.-% Essigsäure, 0,01 bis 2 Gew.-% Propionsäure, 0,05 bis 1 Gew.-% Formaldehyd, 0,05 bis 2 Gew.-% Aldehyde, 0,01 bis 0,5 Gew.-% der Summe aus Maleinsäure und Maleinsäureanhydrid und 20 bis 98 Gew.-%, vorzugsweise 50 bis 98 Gew.-% inerte Verdünnungsgase. Als inerte Verdünnungsgase sind insbesondere gesättigte C₁-C₆-Kohlenwasserstoffe, wie 0 bis 95 Gew.-% Methan und/oder Propan, daneben 1 bis 30 Gew.-% Wasserdampf, 0,05 bis 15 Gew.-% Kohlenoxide und 0 bis 95 Gew.-% Stickstoff, jeweils bezogen auf 100 Gew.-% Reaktionsgas, enthalten.

Die Verfahrensstufen zur Abtrennung der Acrylsäure aus dem Reaktionsgemisch werden im folgenden beschrieben:

### Stufe I

Das heiße Reaktionsgas wird durch Teilverdampfen des Lösungsmittels in einem Direktkondensator oder Quenchapparat, vor der Absorption abgekühlt. Hierfür eignen sich insbesondere Venturiwäscher, Blasensäulen oder Sprühkondensatoren. Dabei kondensieren die schwersiedenden Nebenkomponenten des Reaktionsgases in das nicht verdampfte Lösungsmittel. Außerdem ist die Teilverdampfung des Lösungsmittels ein Reinigungsschritt für das Lösungsmittel. In einer bevorzugten Ausführungsform der Erfindung wird ein Teilstrom des nicht verdampften Lösungsmittels, vorzugsweise 1 bis 10% des der Absorptionskolonne zugeführten Massenstroms, abgezogen und einer Lösungsmittelreinigung unterworfen. Hierbei wird das Lösungsmittel überdestilliert und zurück bleiben die schwersiedenden Nebenkomponenten, die - bei Bedarf weiter eingedickt - entsorgt, z.B. verbrannt, werden können. Diese Lösungsmitteldestillation dient der Vermeidung einer zu hohen Konzentration an Schwersiedem im Lösungsmittelstrom. Das überdestillierte Lösungsmittel wird vorzugsweise dem beladenen Lösungsmittelstrom aus der Absorptionskolonne zugeführt.

### Stufe II

In Stufe II werden die Acrylsäure und ein Teil der Nebenkomponenten aus dem Reaktionsgas durch Absorption in einem hochsiedenden Lösungsmittel abgetrennt.

Vorzugsweise liegt der Siedepunkt des hochsiedenden Lösungsmittels wenigstens 20°C, insbesondere 50°C, stärker bevorzugt 70°C über dem Siedepunkt der Acrylsäure bzw. Methacrylsäure. Bevorzugte Lösungsmittel, wobei in vorliegender Anmeldung der Begriff Lösungsmittel auch Lösungsmittelgemische umfaßt, haben Siedepunkte (bei Normaldruck) von 180 bis 400°C, insbesondere von 220 bis 360°C. Geeignete Lösungsmittel sind hochsiedende, extrem hydrophobe Lösungsmittel, die keine nach außen wirkende polare Gruppe enthalten, wie aliphatische oder aromatische Kohlenwasserstoffe, z.B. Mittelölfraktionen aus der Paraffindestillation, oder Äther mit sperrigen Gruppen am O-Atom, oder Gemische davon, wobei diesen vorteilhafterweise ein polares Lösungsmittel, wie das in DE-A-43 08 087 offenbarte 1,2-Dimethylphthalat, zugesetzt wird. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkane, z.B. 4-Methyl-4'-benzyl-diphenylmethan und dessen Isomere 2-Methyl-2'-benzyl-diphenylmethan, 2-Methyl-4'-benzyl-diphenylmethan und 4-Methyl-2'-benzyl-diphenylmethan und Gemische solcher Isomerer.

Ein besonders bevorzugtes Lösungsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, bezogen auf 100 Gew.-% Diphenyl und Diphenylether, beispielsweise das im Handel erhältliche Diphyl®. Vorzugsweise enthält dieses Lösungsmittelgemisch weiterhin ein polares Lösungmittel wie Dimethylphthalat in einer Menge von 0,1 bis 25 Gew.-%, bezogen auf das gesamte Lösungsmittelgemisch. Dadurch reduziert sich die Verschmutzungsanfälligkeit der Anlagen.

Vorliegend bezeichnen die Begriffe Hoch- oder Schwersieder, Mittelsieder und Leichtsieder sowie entsprechend adjektivisch gebrauchte Begriffe Verbindungen, die einen höheren Siedepunkt als die Acrylsäure besitzen (Hochsieder) bzw. solche, die in etwa den gleichen Siedepunkt wie Acrylsäure besitzen (Mittelsieder) bzw. solche, die einen niedrigeren Siedepunkt als Acrylsäure besitzen (Leichtsieder).

Die Absorption erfolgt in einer Gegenstromabsorptionskolonne, die vorzugsweise mit Fühlkörpern oder strukturierten Packungen bestückt ist, und die von oben mit Lösungsmittel beaufschlagt wird. Das gasförmige Reaktionsprodukt und gegebenenfalls verdampftes Lösungsmittel aus dem Quenchapparat werden von unten in die Kolonne eingeleitet und anschließend auf Absorptionstemperatur abgekühlt. Die Abkühlung erfolgt vorteilhafterweise durch Kühlkreise, d.h. erwärmtes Lösungsmittel wird aus der Kolonne abgezogen, in Wärmetauschern abgekühlt und wieder an einer Stelle oberhalb der Abzugsstelle der Kolonne zugeführt. Nach der Absorption befinden sich alle Schwersieder, der größte Teil der Acrylsäure sowie ein Teil der Leichtsieder im Lösungsmittel.

Das verbleibende, nicht absorbierte Reaktionsgas wird weiter abgekühlt, um den kondensierbaren Teil der leichtsiedenden Nebenkomponenten, insbesondere Wasser, Formaldehyd und Essigsäure, durch Kondensation davon abzutrennen. Dieses Kondensat wird im folgenden Sauerwasser genannt. Der verbleibende Gasstrom besteht überwiegend aus Stickstoff, Kohlenoxiden und nicht umgesetzten Edukten. Vorzugsweise wird dieser teilweise wieder als Verdünnungsgas, im folgenden Kreisgas genannt, den Reaktionsstufen zugeführt. Der Luftstickstoffund ein Teil der nichtkondensierten Nebenkomponenten werden als Abgas ausgeschleust und vorzugsweise verbrannt.

### Stufe III und VI

In Verfahrensstufe III wird die Acrylsäure zusammen mit den mittelsiedenden Komponenten sowie dem letzten Rest an leichtsiedenden Nebenkomponenten vom Lösungsmittel abgetrennt, woher ein erster Teilstrom III A anfällt, der überwiegend Acrylsäure enthält sowie ein zweiter Teilstrom III B, der überwiegend das Lösungsmittel enthält.

In bevorzugter Weise erfolgt die Abtrennung der Acrylsäure aus dem Gemisch mit dem Lösungsmittel durch Teilverdampfung. Die Teilverdampfung wird bei einem Druck zwischen 10 und 200 mbar und entsprechenden Verdampfungstemperaturen von 60° bis 130°C, insbesondere bei einem Druck von 60 bis 100 mbar und Temperaturen von 90° bis 110°C, durchgeführt. Die Energie zum Verdampfen des Teilstromes IIIA wird soweit wie möglich durch Abkühlen des Reaktionsgases gewonnen. Die fehlende Restenergie wird durch Dampfkondensation gedeckt. Als Ergebnis der Verdampfung entsteht ein Brüdenstrom sowie ein Flüssigstrom. Der Brüdenstrom III A enthält den größten Teil der Acrylsäure, d.h. Acrylsäurekonzentrationen von ca. 70 bis 95%, bevorzugt ca. 80 bis 90%. Die Acrylsäure wird daraus in weiteren Verfahrensschritten destillativ gereinigt. Der Flüssigstrom III B aus dem Verdampfer enthält vorwiegend das Lösungsmittel sowie Acrylsäure in einer Konzentration von ca. 5 bis 15 Gew.-%.

Dieser Flüssigstrom III B wird anschließend durch Strippen gereinigt. Die Reinigung des Lösungsmittels wird nachfolgend als Verfahrensstufe IV beschrieben.

Gemäß einer bevorzugten Ausführungsform erfolgt die destillative Gewinnung der (Meth)acrylsäure aus Teilstrom IIIA in folgenden Verfahrensschritten:
- VI-I: Abtrennung eines Reststroms (a), der neben (Meth)acrylsäure die Leichtsieder, sowie einen Teil der Mittelsieder und einen Teil der Schwersieder enthält, sowie eines Teilstroms (b), der vollständig oder nahezu vollständig frei von Leichtsiedern ist und
- VI-II: Gewinnung der (Meth)acrylsäure aus dem Teilstrom (b).

Die Abtrennung der Acrylsäure aus Stom III A (Stufe VI) erfolgt destillativ, wobei grundsätzlich jede Destillationskolonne verwendet werden kann. Vorteilhafterweise wird hierzu eine Kolonne mit Dual-Flow-Böden verwendet.

Der Teilstrom IIIA wird kondensiert und läuft durch die Kolonne VI-I abwärts. Im Gegenzug steigt Dampf, vorwiegend dampfförmige Acrylsäure, aus dem Sumpf nach oben und strippt dabei die Leichtsieder aus der Flüssigkeit, so daß der im Sumpf ankommende Flüssigkeitsstrom (b) nahezu leichtsiederfrei ist. Bei der Strippung bleiben dagegen die Mittelsieder und Schwersieder überwiegend in der Flüssigkeit und reduzieren die Polymerisationsneigung der Acrylsäure während des Strippvorgangs.

Am Kopf der Kolonne wird dann nach einer Partialkondensation ein an Leichtsiedern reicher Strom (a) abgezogen. Da dieser Strom aber noch Acrylsäure enthält, wird der Brüdenstrom aus der Destillationsstufe VI vorteilhafterweise nicht verworfen, sondern kondensiert und abgekühlt, wonach das kalte Kondensat anschließend in die Quenchstufe I oder die Absorbtionsstufe II zurückgefahren wird.

Die bevorzugten Betriebsparameter in der Abtriebskolonne sind:
Kopfdruck < 200 mbar, insbesondere < 100 mbar, besonders bevorzugt < 50 mbar,
Sumpftemperatur < 140°C, insbesondere < 120°C, besonders bevorzugt < 100°C und
Acrylsäurekonzentration im Sumpf 5 bis 15 Gew.-%, besonders bevorzugt 8 bis 12 Gew.-%.

Die Gewinnung der Acrylsäure aus dem Teilstrom (b) erfolgt bevorzugt durch Auftrennen des Teilstroms (b) in einen ersten, Rohacrylsäure enthaltenden Teilstrom, der gegebenenfalls weiter gereinigt werden kann, sowie einen Teilstrom (c). Die Verfahrensstufe VI-II erfolgt bevorzugt destillativ in einer Auftriebskolonne. Bevorzugt haben die Abtriebskolonne für die Verfahrensstufe VI-I und die Auftriebskolonne für die Verfahrensstufe VI-II einen gemeinsamen Sumpf. Der als Ergebnis der Verfahrensstufe VI-I im gemeinsamen Sumpf von Abtriebs- und Auftriebskolonne anfallende Teilstrom (b) wird in Verfahrensstufe VI-II in der Auftriebskolonne aufgetrennt. Dabei fällt im Kolonnensumpf ein Teilstrom (c) an, der vorwiegend das Lösungsmittel enthält und der, gegebenenfalls nach einer Reinigung, insbesondere durch Verdampfung in einem Quench, in die Absorptionsstufe rezirkuliert wird. In der Auftriebskolonne steigt der vollständig oder nahezu vollständig von Leichtsiedern freie Dampf nach oben, wobei die Mittelsieder und Schwersieder durch den flüssigen Rücklauf aus dem Dampf ausgewaschen werden. Am Kolonnenkopf wird der Brüden kondensiert, ein Teil wird am Kopf als Produkt abgezogen, der Rest ist flüssiger Rücklauf. Das Produkt ist Acrylsäure, die weitgehend frei ist von Leichtsiedern, Mittelsiedern und Schwersiedern. Diese Acrylsäure wird Roh-Acrylsäure genannt.

Die in Stufe VI erhaltene Roh-Acrylsäure enthält, jeweils bezogen auf die Roh-Acrylsäure, vorzugsweise 98 bis 99,8 Gew.-%, insbesondere 98,5 bis 99,5 Gew.-% Acrylsäure und 0,2 bis 2 Gew.-%, insbesondere 0,5 bis 1,5 Gew.-% Verunreinigungen, beispielsweise Essigsäure, Aldehyde und Maleinsäureanhydrid. Diese Acrylsäure kann, sofern die Anforderungen an ihre Reinheit nicht sehr hoch sind, gegebenenfalls bereits zur Veresterung verwendet werden.

In einer bevorzugten Ausführungsform der Erfindung wird das Sauerwasser, das noch Acrylsäure gelöst enthalten kann, mit einem kleinen Teilstrom des nahezu Acrylsäure-freien Lösungsmittel (aus Stufe IV) extraktiv behandelt. Der wäßrige Strom aus der Sauerwasserextraktion kann, was insbesondere bei Vorhandensein von Umweltschutzauflagen erforderlich sein kann, eingeengt werden.

Wird eine Acrylsäurekonzentration im Bereich von 5 bis 15 Gew.-%, insbesondere von 8 bis 12 Gew.-% im Sumpfprodukt zugelassen, so wird es möglich, bei einem Sumpfdruck von ca. 250 mbar eine Sumpftemperatur < 155°C einzuhalten, und damit den Verschmutzungsgrad (Foulinggrad) drastisch zu reduzieren.

### Stufe IV und V

Vor der Rückführung in die Absorptionsstufe I muß der Lösungsmittelstrom weitgehend von Acrylsäure abgereinigt werden, um erneut Acrylsäure aus dem Reaktionsgas der Gasphasenoxidation aufnehmen zu können; die Acrylsäurekonzentration im Lösungsmittelstrom soll dabei 1 Gew.-%, bevorzugt 0,5 Gew.-% nicht übersteigen.

Der in Stufe III anfallende Lösungsmittelstrom IIIB kann noch größere Mengen an Acrylsäure, bis etwa 15 Gew.-%, enthalten. Die Abreicherung des Lösungsmittels von Acrylsäure erfolgt durch Strippen mit einem Inertgas oder einem inerten Gasgemisch, bevorzugt mit einem Teilstrom des Kreisgases oder nur Propan, sofern Propan als Verdünnungsgas eingesetzt wird. Das Strippgas ist besonders bevorzugt ein Teilstrom des Kreisgases. Das Strippen erfolgt bei Drücken von etwa 1,1 bis 2,0 bar, bevorzugt bei Drücken von 1,3 bis 1,6 bar sowie bei Temperaturen von ca. 80 bis 120°C, bevorzugt von 110 bis 120°C. Beim Strippen wird der zu reinigende Lösungsmittelstrom am Kopf einer Kolonne aufgegeben; er fließt über die Einbauten in Richtung Sumpf. Im Gegenstrom wird in den Sumpf der Strippkolonne das Strippgas eingeleitet. Während das Strippgas in Richtung Kolonnenkopf strömt, nimmt es Acrylsäure aus dem flüssigen Lösungsmittelstrom auf, so daß aus dem Sumpf der Strippkolonne ein gereinigter Lösungsmittelstrom abgezogen werden kann, der eine Acrylsäurekonzentration von maximal 1 Gew.-%, bevorzugt von maximal 0,5 Gew.-% enthält. Dieses weitgehend acrylsäurefreie Lösungsmittel kann anschließend wieder zur Absorptionsstufe (I) rezirkuliert werden.

Das mit Acrylsäure beladene Strippkreisgas wird zweckmäßigerweise in die Stufe, in der die Teilverdampfung des Lösungsmittels erfolgt, oder in die Absorptionskolonne rezirkuliert.

Bevorzugt wird das Strippkreisgas aus Verfahrensstufe IV in das Reaktionsgas vor Verfahrensstufe II zurückgeführt.

Die Erfindung wird im folgenden anhand einer Zeichnung sowie anhand von Ausführungsbeispielen näher erläutert.

Es zeigen:
Figur 1, die schematische Darstellung einer Anlage nach dem Stand der Technik und
Figur 2, die schematische Darstellung einer Anlage nach der Erfindung.

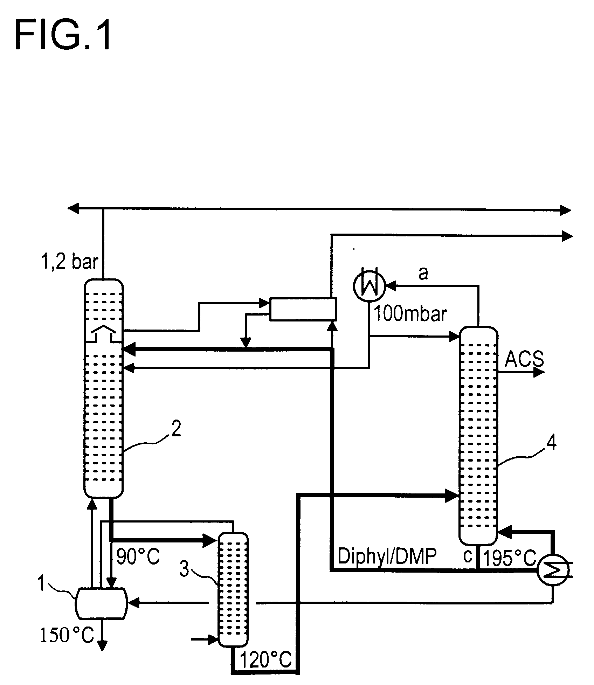

Im folgenden wird zunächst als Vergleichsbeispiel die Gewinnung von Acrylsäure nach dem herkömmlichen Verfahren in einer Anlage entsprechend Figur 1 beschrieben:
ein Gasstrom aus der Gasphasenoxidation zu Acrylsäure von 2900 Nl/h, einer Temperatur von 270°C und einem Druck von 1,6 bar mit den Hauptkomponenten (jeweils in Gew.-%)
Stickstoff (85),
Acrylsäure (10) und
Wasser (4)
wurde in einem Venturiquench 1 durch direkten Kontakt mit im Bereich des engsten Querschnitts des Venturi-Rohres angebrachten Schlitzen eingedüster Quenchflüssigkeit (140 - 150°C) aus 57,4 Gew.-% Diphenylether, 20,7 Gew.-% Diphenyl, 20 Gew.-% o-Dimethylphthalat, Rest andere Komponenten, auf eine Temperatur von 150°C abgekühlt. Anschließend wurde in einem nachgeschalteten Tropfenabscheider (Vorlagebehälter mit oben weggeführtem Gasrohr) der tropfenförmig flüssig gebliebene Anteil der Quenchflüssigkeit von der aus Reaktionsgas und verdampfter Quenchflüssigkeit bestehenden Gasphase abgetrennt und in einem Kreislauf zum Venturiwäscher rückgeführt. Ein Teilstrom der rückgeführten Quenchflüssigkeit wurde dabei einer Lösungsmitteldestillation unterzogen, wobei die Quenchflüssigkeit überdestilliert wurde und schwersiedende Nebenkomponenten, die verbrannt wurden, zurückblieben.
Die eine Temperatur von ca. 150°C aufweisende Gasphase wurde in den unteren Teil einer Füllkörperabsorptionskolonne 2 geführt (3 m hoch; Doppelmantel aus Glas; Innendurchmesser 50 mm; drei Füllkörperzonen der Längen (von unten nach oben) 90 cm, 90 cm und 50 cm; die Füllkörperzonen waren von unten nach oben wie folgt thermostatiert: 90°C, 60°C, 20°C; die vorletzte und die letzte Füllkörperzone waren durch einen Kaminboden getrennt; die Füllkörper waren Metallwendeln aus Edelstahl mit einem Wendeldurchmesser von 5 mm und einer Wendellänge von 5 mm; unmittelbar oberhalb der mittleren Füllkörperzone wurde das Absorptionsmittel zugeführt und dem Gegenstrom von 2900 g/h des gleichfalls aus 57,4 Gew.-% Diphenylether, 20,7 Gew.-% Diphenyl, 20 Gew.-% o-Dimethylphthalat und Rest aus anderen Komponenten zusammengesetzten, mit einer Temperatur von 50°C aufgegebenen Absorptionsmittels, ausgesetzt. Der Ablauf der Absorptionskolonnen der neben Acrylsäure auch leichtsiedende Nebenprodukte wie z.B. Acrolein und Essigsäure absorbiert enthielt, wurde in einem Wärmtauscher indirekt auf 100°C erwärmt und aufden Kopf einer Desorptionkolonne 3 gegeben, die ebenfalls als Füllkörperkolonne einer Länge von 2 m ausgeführt war (Doppelmantel aus Glas; 50 mm Innendurchmesser; Füllkörper: Edelstahlwendeln mit Wendeldurchmesser von 5 mm und Wendellänge von 5 mm; eine Füllkörperzone der Länge 1 m; thermostatiert auf 120°C). In der Desorptionskolonne 3 wurden die im Vergleich zur Acrylsäure leichtersiedenden Komponenten wie Acrolein und Essigsäure durch Strippen mit 600 Nl Luft/h (Gegenstrom; Zuführtemperatur 120°C) weitgehend aus dem Acrylsäure/Absorptionsmittel-Gemisch entfernt. Das die Desorptionskolonne 3 verlassende Strippgas wurde rezirkuliert und mit dem heißen Reaktionsgas der Acroleinoxidationsstufe vor dessen Eintritt in den Venturiquench (Stufe I) vereinigt.

Das in der Absorptionskolonne 2 die zweite Füllkörperzone nach oben verlassende nicht absorbierte Gasgemisch wurde in der dritten Füllkörperzone weiter abgekühlt, um den kondensierbaren Teil der darin enthaltenen Nebenkomponenten z.B. Wasser und Essigsäure, durch Kondensation abzutrennen. Dieses Kondensat wird Sauerwasser genannt. Zur Erhöhung der Trennwirkung wurde ein Teil des Sauerwassers oberhalb der dritten Füllkörperzone der Absorptionkolonne 2 mit einer Temperatur von 20°C in die Absorptionskolonne 2 rückgeführt. Die Entnahme des Sauerwassers erfolgte unterhalb der obersten Füllkörperzone vom dort angebrachten Kaminboden. Das Verhältnis von rückgeführten zu abgezogenem Sauerwasser betrug 200 g/g. Das entnommene Sauerwasser enthielt neben 97,5 Gew.-% Wasser auch noch 0,8 Gew.-% Acrylsäure. Diese kann bei Bedarf wie in der DE-A 196 00 955 beschrieben rückgewonnen werden. 1600 Nl/h des die Absorptionskolonne 2 letzlich verlassenden Gasstromes wurden als Kreisgas in die Propenoxidation rückgeführt. Der Rest wurde verbrannt.

Die Sumpfflüssigkeit der Desorptionkolonne 3 wurde auf den 8. Boden von unten einer 57 Dual-Flow-Böden enthaltenden Bodenkolonne 4 zugeführt (Innendurchmesser: 50 mm; Länge 3,8 m; Kopfdruck: 100 mbar; Sumpfdruck: 280 mbar; Sumpftemperatur: 195°C; auf dem 9. Boden war ein Druckverlustwiderstand angebracht) und in der Bodenkolonne 4 rektifiziert. Am 48. Boden von unten wurde die Roh-Acrylsäure via Seitenabzug entnommen. Die Reinheit der entnommenen Roh-Acrylsäure lag bei ≥ 98 Gew.-%. Am Kopf der Rektifikationkolonne 4 wurde nach einer Partialkondensation (Rücklaufverhältnis 8,7) ein an Leichtsiedern angereicherter, Acrylsäure enthaltender, Gasstrom abgezogen, der oberhalb der untersten Füllkörperzone in die Absorptionskolonne 2 rückgeführt wurde. Aus dem Sumpf der Rektifikationskolonne 4 wurde das an Leichtsieder freie und an Acrylsäure nahezu freie Absorptionmittel abgezogen und oberhalb der zweiten Füllkörperzone (von unten betrachtet) in die Absorptionskolonne 2 rückgeführt. Ein Teilstrom von 50 g/h wurde zur Ausschleusung der schwersiedenden Nebenkomponenten dem Venturiquench 1 zugeführt. Dem Rücklauf am Kopf der Rektifikationskolonne 4 wurde Phenothiazin als Polymerisationsinhibitor zugesetzt und zwar in solchen Mengen, daß der Seitenabzug 300 ppm Phenothiazin enthielt (eine schematische Darstellung des Aufarbeitungsverfahrens des Reaktionsgases der Acroleinoxidationsstufe zeigt die DE-A 196 00 955; darüber hinaus ist die Aufarbeitungsweise auch in der DE-A 43 08 087 dargestellt).

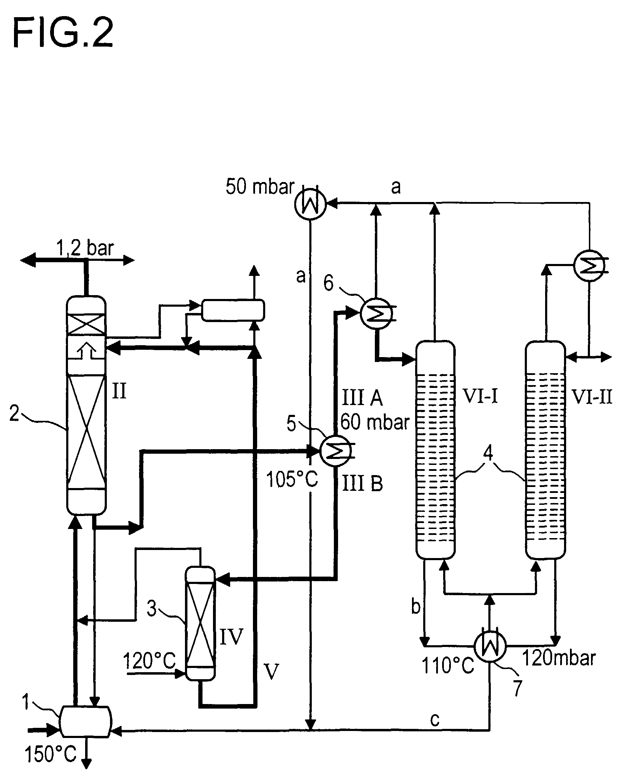

Im folgenden wird ein Ausführungsbeispiel für das erfindungsgemäße Verfahren, in Verbindung mit Figur 2 beschrieben.
Die Verfahrensführung im Venturiquench 1 und in der Absorptionkolonne 2 entspricht dem vorstehend in Verbindung mit Figur 1 beschriebenen Verfahren nach dem Stand der Technik. Abweichend davon wurde der Ablauf der Absorptionkolonne 2 nicht auf den Kopf einer Desorptionskolonne 3, sondern auf einen Zwangsumlaufentspannungsverdampfer 5 gegeben, der bei 60 mbar und 105°C betrieben wurde. Dabei wurde ein mit Acrylsäure beladener Lösungsmittelstrom von 5230 g/h (Hauptkomponenten, jeweils in Gew.-%: Lösungsmittel 61, Acrylsäure 30, Essigsäure 8118ppm, Maleinsäureanhydrid 2000 ppm) in einen ersten Teilstrom IIIA von 2160 g/h, der überwiegend Acrylsäure enthielt (Hauptkomponenten, jeweils in Gew.-% : Lösungsmittel 20, Acrylsäure 77 und Essigsäure 0,22) sowie einen zweiten Teilstrom IIIB von 3070 g/h, der überwiegend das Lösungsmittel enthielt (Hauptkomponenten jeweils in Gew.-% : Lösungsmittel 83, Acrylsäure 5, und Essigsäure 636 ppm) aufgetrennt.

Der Teilstrom IIIB wurde aufden Kopf der Strippkolonne 3 aufgegeben. Es handelte sich dabei um dieselbe Strippkolonne 3 wie im Verfahren nach dem Stand der Technik (Figur 1) sowie um dasselbe Strippgas, d.h. ein Luftstrom von 600 Nl/h. Die Funktion der Stippkolonne 3 war jedoch nach den beiden Verfahren, dem Stand der Technik einerseits und dem erfindungsgemäßen Verfahren andererseits, unterschiedlich: im Verfahren nach dem Stand der Technik (Figur 1) wurde auf den Kopf der Strippkolonne 3 der Ablauf aus der Absorptionkolonne 2 gegeben, die Strippkolonne 3 fungierte als Desorptionkolonne. Demgegenüber wurde beim Verfahren nach der Erfindung (Figur 2) auf den Kopf der Strippkolonne 3 der Teilstrom IIIB aus dem Verdampfer gegeben; die Strippkolonne 3 diente hier zur Abreinigung des Lösungsmittels von Acrylsäure (Verfahrensstufe IV). Das gereinigte Lösungsmittel wurde aus dem Sumpf der Strippkolonne 3 abgezogen und zum Kopf der Absorptionskolonne 2 rezirkuliert.

Der im Verdampfer 5 anfallende Teilstrom IIIA wurde in einem Wärmetauscher 6 bei 100 mbar kondensiert und das Kondensat wurde der, anders als nach dem Stand der Technik, nunmehr am 28. Boden zweigeteilten Rektifikationskolonne 4, und zwar deren Abtriebsteil, zugeführt. Im Abtriebsteil der Rektifikationskolonne 4 fand die Verfahrensstufe VI-I statt, d.h. aus dem Teilstrom IIIA wurden mit Acrylsäuredampf im Gegenstrom die Leichtsieder ausgestrippt, wogegen die Mittelsieder und Schwersieder überwiegend in der Flüssigkeit verblieben. Aus dem Sumpf des Abtriebsteils der Rektifikationskolonne 4 wurde ein nahezu leichtsiederfreier Strom b (Hauptkomponenten in Gew.-% : Lösungsmittel 28, Acrylsäure 71, Essigsäure 721 ppm, Maleinsäureanhydrid 4026 ppm) entnommen. Der Teilstrom b wurde dem gemeinsamen Verdampfer 7 des Abtriebsteils und des Auftriebsteils der Rektifikationskolonne 4 zugeführt, aus dem Verdampfer 7 wurde ein Reststrom c abgezogen (480 g/h, Hauptkomponenten, in Gew.-%: Lösungsmittel 87, Acrylsäure 10, Maleinsäureanhydrid 7000 ppm) und dem Venturiquench 1 zugeführt. Der die Roh-Acrylsäure enthaltende Brüdenstrom aus dem Verdampfer 7 wurde zwecks Gewinnung der Acrylsäure (Verfahrensstufe VI-II) dem Auftriebsteil der Rektifikationskolonne 4 zugeführt und durch den Acrylsäurerücklauf von Mittelsiedern und Schwersiedern gereinigt. Am Kopf des Auftriebsteils der Rektifikationskolonne 4 wurde ein Strom von 420 g/h des Hauptprodukts Acrylsäure abgezogen, der noch 1500 ppm Essigsäure und 50 ppm Maleinsäureanhydrid enthielt. Der Brüden aus dem Abtriebsteil der Rektifikationskolonne 4, dem Apparat in dem die Verfahrensstufe VI-I stattfand, wurde als Reststrom a mit 95 Gew.-% Acrylsäure, 0,8 Gew.-% Lösungsmittel und 3,6 Gew.-% Essigsäure kondensiert und ebenfalls dem Venturiquench 1 zugeführt.

Beim Vergleichsverfahren nach dem Stand der Technik (Figur 1) betrug die Laufzeit der gesamten Anlage 14 Tage. Demgegenüber wurde in der Anlage, die nach dem erfindungsgemäßen Verfahren betrieben wurde (Figur 2) nach einer Betriebsdauer von 24 Tagen praktisch keine Verschmutzung festgestellt, insbesondere auch nicht in der Absorptionskolonne 2 und in der Strippkolonne 3.

## Patentansprüche

1. Verfahren zur kontinuierlichen Gewinnung von (Meth)acrylsäure aus dem (Meth)acrylsäure, Leichtsieder, Mittelsieder und Schwersieder enthaltenden Reaktionsgas einer katalytischen Gasphasenoxidation durch
(I) Quenchen des Reaktionsgases durch Siedekühlung mit einem hochsiedenden organischen Lösungsmittel,
(II) Abtrennung der (Meth)acrylsäure aus dem gequenchten Reaktionsgas durch Absorption in das hochsiedende organische Lösungsmittel,
(III) Auftrennung des mit (Meth)acrylsäure beladenen organischen Lösungsmittels in einen ersten Teilstrom (IIIA), der überwiegend (Meth)acrylsäure enthält sowie in einen zweiten Teilstrom (IIIB), der überwiegend das Lösungsmittel enthält,
dadurch gekennzeichnet, daß
(IV) Teilstrom IIIB mit Inertgas frei an (Meth)acrylsäure gestrippt wird,
(V) das gereinigte Lösungsmittel aus Teilstrom IIIB in die Absorptionsstufe II zurückgeführt wird und daß
(VI) (Meth)acrylsäure aus Teilstrom IIIA destillativ gewonnen wird, wobei alle in Stufe VI anfallenden flüssigen Restströme in die Quenchstufe I zurückgeführt werden.

2. Verfahren nach Anspruch 1, dadurch gekennzeichnet, daß die Temperatur in jeder Verfahrensstufe 155°C, insbesondere 140°C, besonders bevorzugt 120°C, nicht übersteigt.

3. Verfahren nach Anspruch 1 oder 2, dadurch gekennzeichnet, daß die Auftrennung des mit (Meth)acrylsäure beladenen Lösungsmittels in Verfahrensstufe III durch partielle Verdampfung erfolgt.

4. Verfahren nach einem der Ansprüche 1 bis 3, dadurch gekennzeichnet, daß die destillative Gewinnung der (Meth)acrylsäure aus Teilstrom IIIA in folgenden Verfahrensschritten erfolgt:
VI-I Abtrennung eines Reststroms (a), der neben (Meth)acrylsäure alle oder nahezu alle Leichtsieder, einen Teil der Mittelsieder und einen Teil der Schwersieder enthält, sowie eines Teilstroms (b), der überwiegend (Meth)acrylsäure enthält und der vollständig oder nahezu vollständig frei von Leichtsiedern ist und
VI-II Gewinnung der (Meth)acrylsäure aus dem Teilstrom (b).

5. Verfahren nach Anspruch 4, dadurch gekennzeichnet, daß die Verfahrensstufe VI-I in einer Abtriebskolonne und die Verfahrensstufe VI-II in einer Auftriebskolonne erfolgen.

6. Verfahren nach einem der Ansprüche 4 oder 5, dadurch gekennzeichnet, daß in Verfahrensstufe VI-II der Teilstrom (b) in Roh-(Meth)acrylsäure sowie einen Reststrom (c) aufgetrennt wird.

7. Verfahren nach einem der Ansprüche 1 bis 6, dadurch gekennzeichnet, daß das Strippgas aus Verfahrensstufe IV in das Reaktionsgas zur Verfahrensstufe I oder II zurückgeführt wird.

8. Verfahren nach einem der Ansprüche 1 bis 7, dadurch gekennzeichnet, daß der Brüden aus der Destillationsstufe VI-I kondensiert und abgekühlt wird und daß das kalte Kondensat anschließend in die Verfahrensstufe I oder II zurückgefahren wird.

9. Verfahren nach einem der Ansprüche 1 bis 8, dadurch gekennzeichnet, daß die Energie zum Verdampfen des Teilstromes IIIA durch Abkühlen des Reaktionsgases gewonnen wird.

10. Verfahren nach einem der Ansprüche 1 bis 9, dadurch gekennzeichnet, daß das Strippgas ein Teilstrom des Kreisgases ist.
